# EUROPEAN PATENT SPECIFICATION

(11) **EP 3 897 683 B1**
(45) Date of publication and mention of the grant of the patent: **15.11.2023**
(21) Application number: 19831642.4
(22) Date of filing: 16.12.2019
(51) Int. Cl.: A61K 36/185, A61K 8/9789, A61P 17/00, A61P 43/00, A61Q 1/00

(54) **COSMETIC, PHARMACEUTICAL AND NUTRACEUTICAL USE OF AN EXTRACT DERIVED FROM CANNABIS SATIVA CELL CULTURES**
KOSMETISCHE, PHARMAZEUTISCHE UND NUTRAZEUTISCHE VERWENDUNG EINES AUS CANNABIS-SATIVA-ZELLKULTUREN GEWONNENEN EXTRAKTS
UTILISATION COSMÉTIQUE, PHARMACEUTIQUE ET NUTRACEUTIQUE D'UN EXTRAIT ISSU DE CULTURES CELLULAIRES DE CANNABIS SATIVA

(30) Priority: 20.12.2018 IT 201800020590
(43) Date of publication of application: 27.10.2021
(73) Proprietor: Arterra Bioscience S.p.A., 80142 Napoli (IT)
(72) Inventor: BIMONTE, Marida, 80142 Napoli (IT); TORTORA, Assunta, 80142 Napoli (IT); APONE, Fabio, 80142 Napoli (IT); COLUCCI, Maria Gabriella, 80142 Napoli (IT)
(74) Representative: Botti & Ferrari S.p.A.
(86) International application number: PCT/EP2019/085357
(87) International publication number: WO 2020/127056

(56) References cited:
- DVORY NAMDAR ET AL: "Medical Cannabis for the Treatment of Inflammation", NATURAL PRODUCT COMMUNICATIONS, vol. 13, no. 3, 1 March 2018 (2018-03-01), XP055614591, US ISSN: 1934-578X, DOI: 10.1177/1934578X1801300304
- Sayed Hussein Farag: "Cannabinoids production in Cannabis sativa L.: An in vitro approach", Dissertation, 26 November 2014 (2014-11-26), pages 1-146, XP055564086, Retrieved from the Internet: URL:https://eldorado.tu-dortmund.de/bitstr eam/2003/34350/1/Dissertation.pdf [retrieved on 2019-03-04]
- RICHARDSON J D ET AL: "CANNABINOIDS REDUCE HYPERALGESIA AND INFLAMMATION VIA INTERACTION WITH PERIPHERAL CB1 RECEPTORS", PAIN, ELSEVIER SCIENCE PUBLISHERS, AMSTERDAM, NL, vol. 75, no. 1, 1 March 1998 (1998-03-01), pages 111-119, XP001027871, ISSN: 0304-3959, DOI: 10.1016/S0304-3959(97)00213-3
- D.C. HAMMELL ET AL: "Transdermal cannabidiol reduces inflammation and pain-related behaviours in a rat model of arthritis", EUROPEAN JOURNAL OF PAIN, vol. 20, no. 6, 1 July 2016 (2016-07-01), pages 936-948, XP055614989, GB ISSN: 1090-3801, DOI: 10.1002/ejp.818
- MATTHIAS A ENGEL ET AL: "Inhibitory CB1 and activating/desensitizing TRPV1-mediated cannabinoid actions on CGRP release in rodent skin", NEUROPEPTIDES, CHURCHILL LIVINGSTONE, GB, vol. 45, no. 3, 26 March 2011 (2011-03-26) , pages 229-237, XP028384894, ISSN: 0143-4179, DOI: 10.1016/J.NPEP.2011.03.005 [retrieved on 2011-04-02]

## Description

### Field of the invention

The present invention refers in general terms to the use in the cosmetic, pharmaceutical and nutraceutical fields of an extract derived from plant cell cultures, to the process for the production of this extract and to the related cosmetic, pharmaceutical and nutraceutical compositions and formulations comprising it, for the use in the various application fields mentioned above.

### Prior art

*Cannabis sativa* is a plant belonging to the genus *Cannabis* used in traditional medicine and as fiber source. It is known that this plant is particularly rich in phytochemicals, including cannabinoids, terpenes and phenolic compounds.

Although the phytocannabinoids produced by this plant have a chemical structure different from that of the endocannabinoids, which are produced in humans, they share the ability to bind the same receptors and to regulate a variety of physiological and pathological processes, such as pain, inflammation, nutrition, mood and memory. Up to now, over 90 phytocannabinoids have been characterized in the plant, among those some with psychotropic effects have been identified, such as THC (tetrahydrocannabinol) and CBN (Cannabinol), and molecules with weak or no psychotropic effect, such as CBD (Cannabidiol) and CBG (Cannabigerol) .

It is also known that most of the effects produced by cannabinoids appear to be dose-dependent: for example, at low doses they are able to reduce the release of a neuropeptide by sensory neurons, namely CGRP ("Calcitonin Gene Related Peptide", a peptide related to the calcitonin gene), while at high doses they stimulate its release.

At high doses of cannabinoids, this peptide, once bound to its receptors, determines the production of proinflammatory cytokines and histamine, and therefore the activation of the signals that lead to inflammation. In contrast, low doses of cannabinoids produce an anti-inflammatory effect by reducing peptide release.

It is also known that the effect of cannabinoids on neuronal activity is dose-dependent: at low doses, they stimulate the production of dopamine, a neurotransmitter that plays an important role in the central nervous system, motor control, cognitive processes and behavior; instead, at high doses, these compounds inhibit neuronal activity.

Cannabinoids are also able to regulate the sensation of pain, as they can stimulate the release of endorphin, a neuropeptide that causes an analgesic effect and a feeling of euphoria by binding to opioid receptors.

It is also known that also other molecules present in *Cannabis* plants have important physiological functions: flavonoids have antioxidant, anti-inflammatory, anti-cancer, anti-obesity and neuro-protective properties, while terpenes exhibit powerful anti-tumor, anxiolytic, analgesic and immune-stimulating properties.

Legislative decree 242 of 2 December 2016, which entered into force on 14 January 2017, gave provisions to promote the cultivation and agri-industrial supply chain of *Cannabis* in Italy, defining 64 different varieties of *Cannabis sativa* as legal, because they contained low levels of THC (less than 0.2%), from which it was possible to obtain semi-finished products, food products and cosmetic ingredients.

Extracts from *Cannabis sativa* plant are already used and patented for various purposes.

The patent application WO 2017/175126 describes a topical composition of a mixture containing a *Cannabis sativa* plant extract (obtained from flower, stem or leaf) and a *Calendula Officinalis* flower extract with anti-aging, moisturizing and solar radiation protection properties.

Patent application CN 105998195 describes the cosmetic use of *Cannabis* leaf extract or seed oil with anti-inflammatory properties and skin sensitivity reduction.

The patent application WO 2010/ 150245 describes a cosmetic and/or pharmaceutical composition containing an extract of *Cannabis* flowers or seeds with anti-inflammatory properties.

The patent application CN 105943613 describes the use of the *Cannabis sativa* whole extract to prevent cardiovascular and cerebrovascular diseases.

The scientific article "Medical Cannabis for the treatment of inflammation", by Dvory Namdar et al. ("Natural product Communications", vol. 13, n.3, 1 March 2018) discloses the use of *Cannabis sativa,* which contains cannabinoids (THC and CBD), in the treatment of inflammatory neuronal diseases (IND), including Parkinson and Alzheimer's diseases.

However, the production of these extracts has several disadvantages.

First, these extracts are obtained from cultivated plants, which are subject to varying environmental and seasonal conditions that not only may compromise the plant growth , but, above all, they can significantly affect the production of secondary metabolites, thus determining a variable content of cannabinoids in the extract, which, as mentioned above, directly influence the biological properties of the extract itself.

In addition, as these extracts are obtained from cultivated plants, they can be contaminated by toxic substances, such as pesticides and/or fertilizers to which plants are exposed during their growth.

Moreover, the extracts may contain pathogens, which inhabit the plant, reducing the quality of the final extract and requiring additional purification steps to obtain the final product suitable for cosmetic, pharmaceutical and nutraceutical applications.

Finally, CN 105998195 and WO 2010150245 describe extracts obtained from *Cannabis* seeds that are particularly rich in lectins, substances that cause important and pathological reactions of the immune system and inducing a destabilization of the immune system and a possible onset of diseases related to impaired immunity.

In the publication "Cannabinoids production in Cannabis sativa L.: An in vitro approach", S. H. Farag (2014), *in vitro* organogenesis from calls cultures and the production of THC and other important cannabinoids in cell suspension, hairy root and trichome cultures of *Cannabis* was achieved, and the regeneration of complete plant via organogenesis from callus cultures was established.

This publication also describes the process for preparing said *Cannabis sativa* extracts, comprising: establishing cell suspension cultures from callus cultures, homogenizing the dry cell suspension with 70% methanol, filtering the extracted solution, and evaporating the filtered extracted solution. The author also explains how to enrich the above extracts with cannabinoids and THCs, but it does not report the presence of other active compounds in the extract nor applications of the mentioned compounds. The THCs were detected at the levels of 8.12 and 4.45 pg/g dry weight, while the most pronounced yield of CBD was 1.5 pg/g dry weight.

The objective technical problem underlying the present invention is to provide a *Cannabis* extract rich in secondary metabolites, suitable for therapeutic and cosmetic uses, and which is essentially free of the drawbacks of the *Cannabis* extracts of the aforementioned known prior art.

### Summary of the invention

In one aspect of the present invention, the aforementioned technical problem is solved by providing an extract derived from *Cannabis sativa* cell cultures for use in the prevention and treatment of inflammation, in particular neuroinflammation, wherein said extract is obtained by a process comprising the steps of:
a) homogenizing the cell cultures in a hydro-alcoholic solution, said hydro-alcoholic solution being an ethanol solution in water with a concentration of ethanol of from 10% to 98% v/v, thus obtaining a homogenate;
b) separating the solid fraction from the liquid fraction of said homogenate, preferably by centrifugation or filtration;
c) bringing the liquid fraction to dryness, thus obtaining said extract.

Preferably, *Cannabis sativa* cells belong to a variety selected from the group comprising *Carmagnola, Santhica 27, Eletta campana* or *Felina 32,* most preferably to the *Carmagnola* variety.

In another aspect thereof, the present invention also relates to a cosmetic use of an extract derived from *Cannabis sativa* cell cultures, preferably belonging to a variety selected from the group comprising Carmagnola, *Santhica 27, Eletta campana* or *Felina 32,* more preferably to the *Carmagnola* variety, said extract being obtained by a process comprising the steps of:
a) homogenizing the cell cultures in a hydro-alcoholic solution, said hydro-alcoholic solution being an ethanol solution in water with a concentration of ethanol of from 10% to 98% v/v, thus obtaining a homogenate;
b) separating the solid fraction from the liquid fraction of said homogenate, preferably by centrifugation or filtration;
c) bringing the liquid fraction to dryness, thus obtaining said extract.

In particular, the cosmetic use is for the treatment of skin blemishes.

"Skin" means skin, hair and nails.

In another aspect, the invention also relates to a process for the preparation of an extract derived from a *Cannabis sativa* cell culture, preferably belonging to a variety selected from the group comprising *Carmagnola, Santhica 27, Eletta campana* or *Felina 32,* more preferably to the *Carmagnola* variety, comprising the steps of:
a) homogenizing the cell culture in a hydro-alcoholic solution, said hydro-alcoholic solution being an ethanol solution in water with a concentration of ethanol of from 10% to 98% v/v, thus obtaining a homogenate;
b) separating the solid fraction from the liquid fraction of said homogenate, preferably by centrifugation or filtration;
c) bringing the liquid fraction to dryness, thus obtaining said extract.

The liquid fraction in the afore-mentioned step c) may be brought to dryness by either evaporation or lyophilization.

Preferably, the *Cannabis sativa* cell cultures are obtained by cutting out vegetable tissues from *Cannabis sativa* plants of a variety selected from *Carmagnola, Santhica 27, Eletta campana* or *Felina 32,* more preferably *Carmagnola* variety, inducing the formation of calli from the tissues on a solid substrate, taking the calli and setting up liquid cell cultures with them.

Preferably, the ratio between the volume of the hydro-alcoholic solution and the weight of the culture of *Cannabis sativa* cells is comprised between 5:1 and 2: 1, advantageously 3:1.

The present invention also relates to a composition comprising an extract derived from a culture of *Cannabis sativa* cells of a variety selected from *Carmagnola, Santhica 27, Eletta campana* or *Felina 32,* preferably *Carmagnola* variety, obtained by the above process, and at least a hydrophilic solvent selected from the group comprising water and saline aqueous solutions, or at least an organic solvent selected from the group comprising oils, alcohols, glycerol, organic acids, amides, amines, aldehydes and ketones.

In one embodiment, the composition comprises between 0.0001 and 10%, preferably between 0.002 and 0.01%, of said extract from *Cannabis sativa* cell cultures, by weight on the total weight of the composition.

In a further aspect thereof, the present invention relates to a composition as defined above for use in the prevention and treatment of inflammation, in particular neuronal inflammation, more specifically that induced by the Calcitonin Gene Related Peptide (CGRP), produced by neuronal cells, and for stimulating the production of neurotransmitters and/or neuropeptides, such as dopamine and endorphin.

In a further aspect thereof, the invention relates to the cosmetic use of a composition as defined above.

The present invention further relates to a pharmaceutical formulation comprising an extract or a composition as defined above, and a pharmaceutically acceptable vehicle for use in the treatment of inflammation, in particular neuronal inflammation, more specifically that induced by the Calcitonin Gene Related Peptide (CGRP) produced by neuronal cells.

Furthermore, the present invention relates to a cosmetic formulation comprising an extract or a composition as defined above, and a cosmetically acceptable vehicle.

The cosmetic formulation can be for example in the form of a cream, gel, lotion for the skin application, lipstick, foundation creams and make-up.

Finally, the present invention relates to a formulation of dietary supplement comprising an extract or a composition for the use as defined above, and a vehicle acceptable from the nutraceutical point of view.

The pharmaceutical and/or food supplement formulation can be for example in the form of oral preparations, such as tablets, capsules, drinks, suspensions or powders.

Advantageously, the Applicant has identified an extract derived from a culture of *Cannabis sativa* cells, in particular a hydro-alcoholic extract, which, by acting through molecular mechanisms, is capable of preventing and/or treating "neurogenic inflammation" and improving neuronal functions by stimulating the production of neurotransmitters and neuropeptides, such as dopamine and endorphins.

The term "neurogenic inflammation", as used herein, indicates inflammation derived from localized release of inflammatory mediators, for example, the CGRP neuropeptide, from afferent neurons. In particular, once released, the neuropeptide induces the release of pro-inflammatory cytokines and histamine from adjacent cells, such as, for example, skin cells and mast cells. Neurogenic inflammation plays an important role in the pathogenesis of numerous skin diseases, including skin hypersensitivity, psoriasis, eczema, rosacea, vitiligo, but also diseases related to other organs and tissues, such as migraine, asthma, fibromyalgia, vasomotor rhinitis and dystonia.

Since this extract is obtained from *in vitro* plant cell cultures, it is free of any toxic contaminating substance (for example, pesticides and/or fertilizers) and environmental pathogens, and is not affected by changes in the seasons and environmental conditions, as the cells grow in controlled laboratory conditions.

Thanks to these conditions, a further important advantage of the present invention is that the characteristics of the plant extracts are standardized, since the production of secondary metabolites is always the same and constant, always guaranteeing the same qualitative characteristics of the product obtained and the same properties.

Another advantage of the present invention is that the culture cells are totipotent and, therefore, they are capable of expressing a wider variety of compounds, in particular secondary metabolites, compared to that present in a differentiated tissue, and they can be manipulated in laboratory. Consequently, the synthesis of such secondary metabolites can be easily modified by physical treatments (visible light, UV, heat, cold) or chemical treatments (sugars, amino acids, phyto-hormones, compounds with oxidizing activity) of cell cultures, thus controlling the production of these metabolites; the extract thus obtained has got a cannabinoid content in sufficient quantities to preserve the activities described in the present invention.

In particular, to ensure that the extract displays the described characteristics and produces an optimal inhibitory effect on the CGRP neuropeptide, the levels of THC and CBN, are preferably comprised between 0.1 and 0.005 µg /g of extract dry weight.

Finally, the extracts obtained from the cells are free of allergenic or irritating substances (such as lectins, which are present, for example, in the seeds of numerous plants) and do not present any potential risk of allergic reaction or hypersensitivity in individuals.

### Brief description of the figures

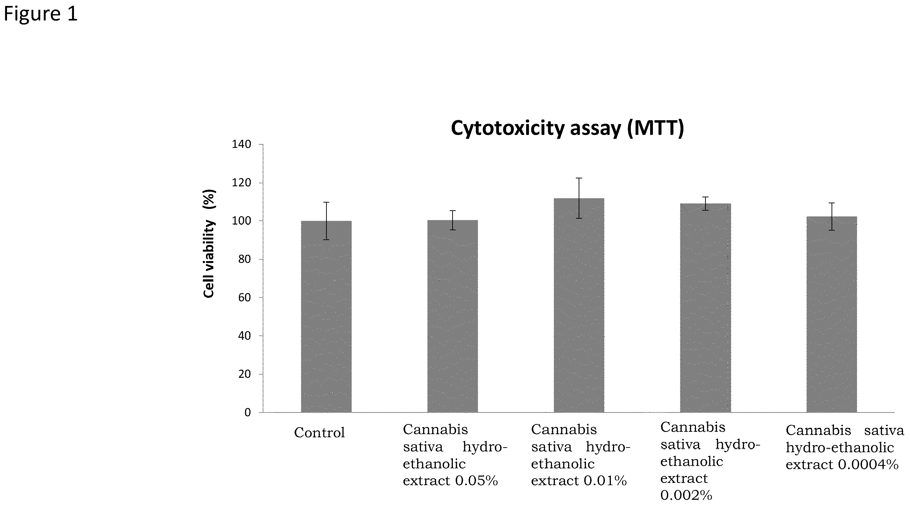

Figure 1 shows a bar graph reporting the results of the cytotoxicity assay (MTT), indicating that the hydro-alcoholic extract according to the invention has no toxic effect on the skin cells at concentrations equal to or less than 0.05% (0.5 mg/ml). The number of viable cells expressed as a percentage of the control (equal to 100%) is shown on the Y-axis.

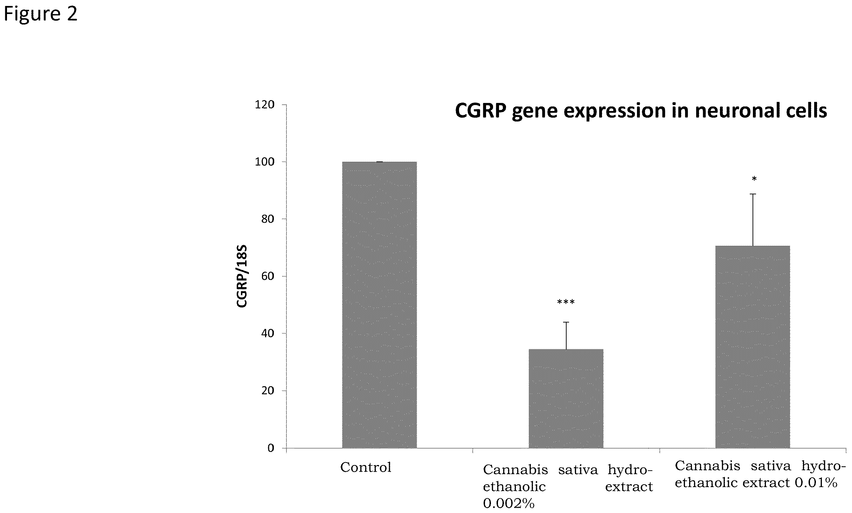

Figure 2 shows a bar graph representing the effect of *Cannabis sativa* extract on the expression of the Calcitonin Gene Related Peptide (CGRP) in neuronal cells; the values in the graph are expressed as percentages with respect to the control cells not treated with the extract (arbitrarily set as 100%)

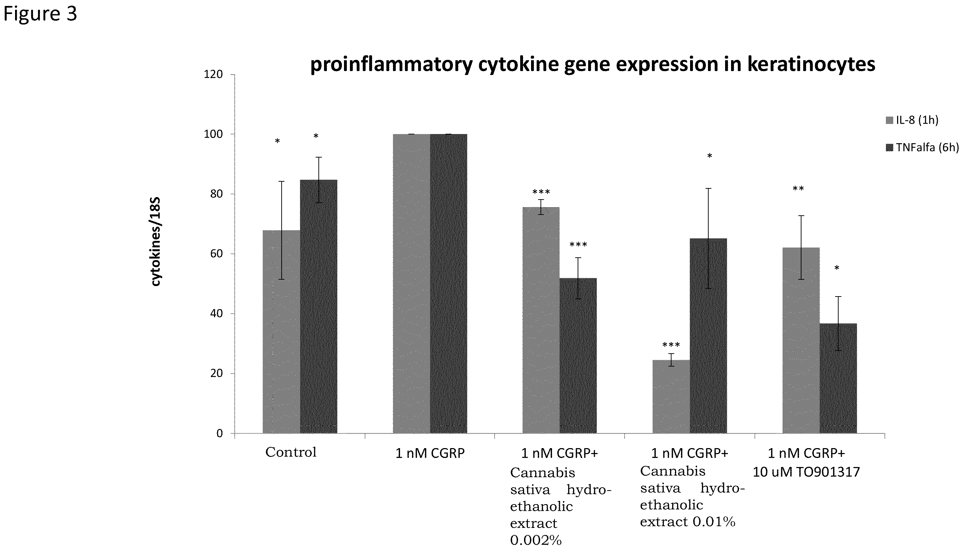

Figure 3 shows a bar graph representing the effect of *Cannabis sativa* extract on the expression of pro-inflammatory cytokines, IL-8 and TNF-alpha induced by CGRP in epidermal cells; the values shown in the graph are expressed as percentages with respect to the stressed cells with the CGRP peptide, arbitrarily set as 100%.

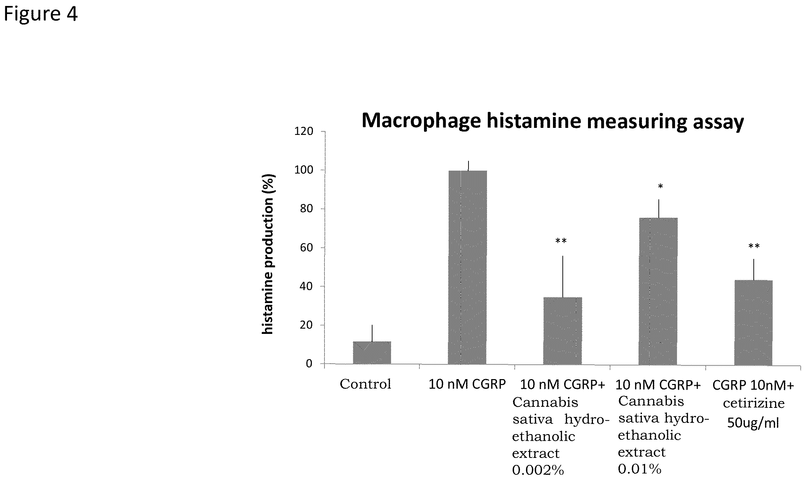

Figure 4 shows a bar graph representing the effect of *Cannabis sativa* extract on histamine production, induced by CGRP, in macrophages; the values shown in the graph are expressed as percentages with respect to the control sample in the presence of the CGRP peptide (stress condition), arbitrarily set as 100%.

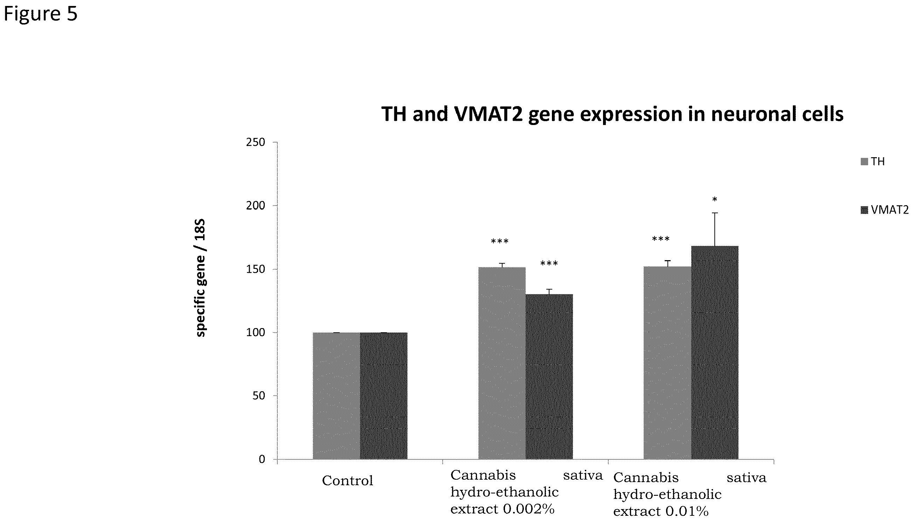

Figure 5 shows a bar graph representing the effect of *Cannabis sativa* extract on the expression of the enzyme responsible for dopamine synthesis, tyrosine hydroxylase (TH), and on the expression of the Vesicular MonoAmine Transporter 2 (VMAT2), which transports dopamine in neuronal cells; the values shown in the graph are expressed as percentages with respect to control cells not treated with the extract (arbitrarily set as 100%).

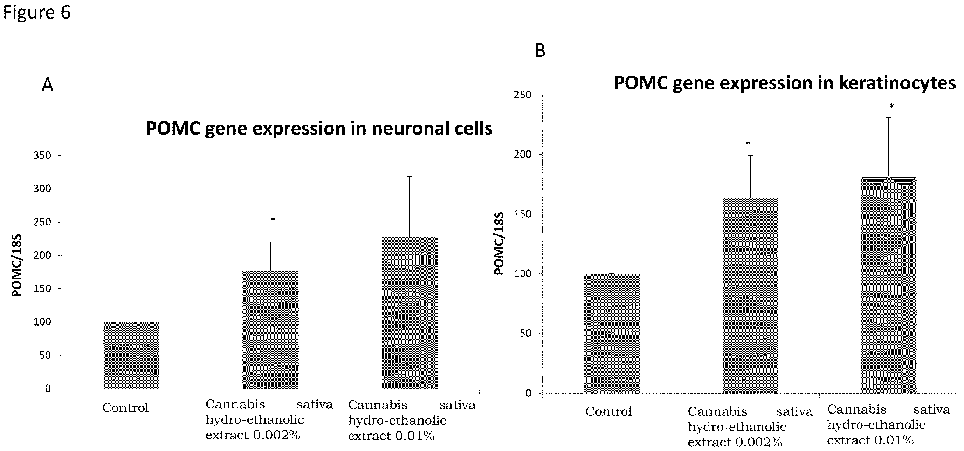

Figure 6 shows a bar graph representing the effect of *Cannabis sativa* extract on the expression of the beta endorphin precursor (POMC) in neuronal cells (panel A) and in human epidermis cells (panel B): the values shown in the graph are expressed as percentages with respect to the control sample arbitrarily set as 100%.

### Detailed description

The Applicant has found of particular interest a hydro-alcoholic extract from plant cell cultures of *Cannabis sativa* for the cosmetic, pharmacological and nutraceutical fields, which is active in reducing inflammation, in particular that induced by the Calcitonin Gene Related Peptide (CGRP) produced by neuronal cells, and in stimulating the production of neurotransmitters, such as dopamine and endorphins.

In particular, this extract is surprisingly able to reduce the production of the neuropeptide CGRP, produced by sensory neurons and which, acting on adjacent cells, such as keratinocytes and macrophages, triggered the production of signals leading to neurogenic inflammation and histamine production.

Surprisingly, this extract is also capable of reducing the production of pro-inflammatory cytokines, such as IL-8 and TNF-α, induced by the neuropeptide on epidermal cells, showing a dual effect: inhibiting the production of the CGRP neurogenic peptide and treating the inflammation triggered by it. In addition, it is able to effectively counteract the production of histamine in the immune system cells, induced by the neurogenic peptide.

In neuronal cells, this extract stimulates the expression of the enzyme responsible for the synthesis of dopamine, which is a precursor of other important neurotransmitters such as norepinephrine (noradrenaline) and epinephrine (adrenaline).

Moreover, this extract induces the expression of the VMAT2 transporter, responsible for the transport at presynaptic level of some important neurotransmitters, such as dopamine, noradrenaline, adrenaline and serotonin from the cytoplasm, where they are synthesized, to vesicles.

According to the present invention, the hydroalcoholic extract described herein is therefore able to stimulate the synthesis and transport of important neurotransmitters, which performed fundamental functions in the nervous system.

Finally, it has been observed that the extract of the present invention stimulates the expression of the POMC gene, precursor of various neurohormones and neuropeptides, including beta endorphins, both in neuronal cells and in human epidermis cells. Indeed, endorphins bind to opioid receptors and cause an analgesic and well-being effect on the human body.

Although endorphins are mainly produced in the central nervous system, a fully functional beta-endorphin/ receptor system is also present in skin cells, and the extract described in the present invention is capable of stimulating the expression of the precursor of the endorphins in both neuronal and human epidermis cells.

Regarding the cosmetic sector, the Applicant has surprisingly found that the extract of the present invention is particularly suitable for the treatment of skin blemishes.

Advantageously, the cell cultures of the invention, obtained by the "plant tissue culture" method described below, show the ability to synthesize a class of secondary metabolites in a controlled and defined way, which were able to regulate important physiological functions in different cell types, including neuronal cells.

Cell cultures are obtained from *Cannabis sativa* leaf disks by inducing the formation of calluses (or "calli") on a solid substrate. The thus-obtained cells are prepared and grown in liquid media that can be subjected to treatments with chemical compounds (for example, salts, sugars, amino acids, vitamins, antioxidants, phytohormones) and/or physical factors (for example, visible light, UV, heat, cold, osmotic stress) to boost the production of the secondary metabolites of interest and increase the biological activity of the derived extracts.

The cells of the thus-obtained cultures are then subjected to mechanical rupture by homogenization in a hydro-alcoholic solution, preferably hydro-ethanolic, in order to obtain a homogenate.

The term "homogenization" means a fragmentation treatment of the plant material, which takes place in a suitable container, such as a ceramic mortar and a pestle previously cooled; metal containers with steel blades can be employed for larger volumes, industrial blenders or presses.

The homogenate is then separated into a liquid fraction and a solid residue by centrifugation and/or filtration of the homogenate. In the embodiment wherein said separation is carried out by centrifugation, the liquid fraction is the supernatant of the obtained centrifugate.

Subsequently, the solvents (ethanol and water) are eliminated from the liquid fraction by roto-evaporation, and/or freeze-drying, drying chambers or spray dryer.

Cosmetic compositions comprising the extract according to the present invention, possibly along with cosmetically acceptable vehicles, solvents, excipients and/or adjuvants are also included in the object of the present invention. These compositions may be in form of gel, creams, cosmetic lotions for application to skin.

Pharmaceutical and nutraceutical compositions comprising the extract of the present invention, possibly along with acceptable vehicles, solvents, excipients e/or adjuvants are also included in the object of the invention. These compositions may be in form of oral preparations, such as tablets, capsules, suspensions or powders.

Preferably, the solvent is a hydrophilic solvent, more preferably selected from water and saline aqueous solutions, or at least a cosmetically acceptable organic solvent, more preferably selected from oils, alcohols, glycerol, organic acids, amides, amines, aldehydes or ketones, or a combination of one or more type of solvent, if they are mixable among them.

The present invention also refers to the cosmetic, pharmaceutical and nutraceutical use of the above-mentioned composition for treating neuroinflammation and stimulating neurotransmitters and neuropeptides.

These compositions represent the raw material to be added to formulations for the preparation of cosmetic or dermatological products, pharmaceutical or nutraceutical preparations suitable for applications.

By way of non-limiting examples of the present invention, below there are examples relating to the preparation of the plant cell culture extract derived from *Cannabis sativa* species, *Carmagnola* variety. Moreover, it is also described an example for preparing an extract according to the present invention, as well as experimental tests demonstrating the biological activity of this extract in the above-mentioned several application fields.

### EXAMPLES

The extract of the present invention was obtained by cell cultures of *Cannabis sativa, Carmagnola* variety, according to the methods described below.

### EXAMPLE 1A,

### Step of callus formation:

To obtain the calluses, leaves of *Cannabis sativa* seedlings, *Carmagnola* variety, were used. The leaves were cut, wound on the surface and cut to obtain pieces of 5 mm x 5 mm size. The pieces of leaf were placed on the solid substrate Gamborg B5 (Duchefa Biochemie), pH 5.7, supplemented with myo-inositol 500 mg /L, 2,4-dichlorophenoxyacetic acid (2.4D) 1mg / L, kinetin 0.01 mg / L, adenine 1mg / L, sucrose 30 g / L and "plant agar" 8 g / L (Duchefa Biochemie), and grown at 26 ° C in the dark. After about 4 weeks of growth the first calluses were obtained on solid culture medium which were subsequently propagated.

### Step of setting up and growth of liquid cultures

Once reached a size of about 2 cm (weight of about 50 mg), the calluses were taken and placed in 150 mL flasks, containing 50 ml of Gamborg B5 culture medium (composition of the solid medium described above but without agar and with kinetin at 0.1 mg/L). The flasks were placed in the dark at a temperature of 26 ° C on a shaker with 120 rpm orbital shaking. After about 4 weeks the necessary density was reached to carry out the cultivation scale up.

### Step of cell collection

The cells were collected through low-porosity filters (20-25 microns), in order to remove the culture medium. The cells were subsequently washed in sterile distilled water and frozen at -20 ° C.

### Preparation of the hydroalcoholic extract

C. *sativa* cells (100 g) were suspended in cold (4 ° C) ethanol 96% in_a ratio of 3:1 volume/weight (approximately 300 ml of ethanol per 100 g of fresh cells) and homogenized in a mechanical blender for 15 minutes.

Once a homogeneous lysate was obtained, the lysate was left in a beaker under stirring for 2 hours, then centrifuged at 4000 g for about 15 minutes at 4 ° C and filtered to remove further residues and recover the supernatant. The supernatant obtained from the filtration (about 300 mL and consisting of ethanol and water) was distilled through under-vacuum evaporation with the aid of a rotary evaporator and, after removing the solvent, about 2.1 g of concentrated extract was obtained.

To perform the biological assays, the extract thus obtained was diluted in ethanol at a concentration ranging between 10% and 0.1%.

The extract was tested on human cells (keratinocytes, HaCat) to establish the doses of use and the cytotoxicity. The extracts were then used in different cellular assays to assess their properties on neurons, skin cells and immune system cells.

### EXAMPLE 1B:

An alternative method for the preparation of the extract according to the present invention corresponds to the above Example 1A, except that, during the 4 weeks in which the liquid cultures grow, the *Cannabis sativa* cells were subjected to cycles of 16 h visible light and 8 h dark, in the presence of 3µg/mL proline.

With respect to Example 1A, an increased cell growth rate and an accelerated biomass recovery were obtained.

### EXAMPLE 2 - Cytotoxicity assay.

In order to determine the concentrations of the extracts to be used for the subsequent assays, a cytotoxicity assay was conducted to determine the range of concentrations in which the extract of the present invention was not harmful to the cells.

This assay is based on the use of the compound MTT [3- (4,5-dimethylthiazolyl) -2,5-diphenyltetrazolium-bromide], first described by Mosmann in 1983. It is based on the ability of the mitochondrial enzyme dehydrogenase of vital cells to hydrolyze the tetrazole ring of MTT (light yellow) and form formazan crystals (dark blue). These crystals are impermeable to cell membranes and accumulate in the cytoplasm of metabolically active cells. The number of living and healthy cells is thus directly proportional to the amount of produced formazan.

HaCat cells, in initial number of 1 × 10⁵ per well, were grown in 96-well plates in DMEM (Dulbecco's Modified Eagle Medium) (Lonza) culture medium, supplemented with 10% fetal bovine serum, for about 8 hours.

After treatment with increasing concentrations of the extracts ranging from 0.05% to 0.0004% (500 µg/ml and 4 µg/ml) for about 48 h, the cells were washed in PBS and incubated with 100 µl of "reaction buffer" per well containing: 10 mM of Hepes, 1.3 mM CaCl₂, 1 mM MgSO₄, 5 mM glucose and 0.5 mg/ml colorimetric substrate MTT in PBS buffer at pH 7.4. After 3 h of incubation at 37 ° C and with 5% of CO₂, 100 µl of solubilizing solution containing 10% of Triton X-100 and 0.1 N of HCl in absolute isopropanol were added to each well. After 2 h, the colorimetric reaction was measured at 595 nm with the Victor3 plate reader.

As shown in Figure 1, the extracts had no toxic effect on the cells at all the used concentrations.

### EXAMPLE 3 - Analysis of cannabinoid content in the Cannabis sativa extract.

In order to determine the cannabinoid content in the C. *sativa* extract, chemical analyses were conducted by a gas chromatography-mass spectrometry (GC/MS) method. GC/MS is an analytical technique that allows to identify and quantify a variety of organic substances in different types of matrices. The chemical analyses, conducted at the Department of Chemical Sciences of the Federico II University of Naples, revealed the presence of Cannabidiol (CBN) and Δ-Tetrahydrocannabidiol (Δ-THC) at concentrations around 1mg/ 100 g (0.01 µg/g) of dry extract, which are values within the legal limits and which define the properties and effectiveness of the extract object of the present invention.

### EXAMPLE 4 - Expression analysis of the neuropeptide Calcitonin Gene Related Peptide (CGRP) in neuronal cells.

In order to analyze the expression of the neuropeptide CGRP in neuronal cells, semi-quantitative RT-PCR ("Reverse transcriptase-polymerase chain reaction") experiments were performed to measure its expression. Neuroblastoma cells (SHSY5Y) in an initial number of 15 × 10⁴ per well were grown in 6-well plates in DMEM culture medium (Lonza), supplemented with 10% FBS for 16 h. The cells were subsequently treated with two different concentrations of the C. *sativa* extract (0.002% and 0.01% w/v) for 6 h.

The cells were then collected, and the RNA was extracted using a kit purchased from Sigma (GenEluteTM Mammalian Total RNA Miniprep Kit). The RNA samples were subjected to a treatment with DNase I (Ambion) to eliminate the contaminating genomic DNA. 2 µl of each sample was loaded on 1% agarose gel in the presence of the "loading dye" (0.25% bromophenol blue; 0.25% xylene cyanol FF; 40% sucrose in water) and quantified using a specific marker for RNA as reference (Riboruler RNA ladder, Fermentas). For quantification, the Gene tools software (Perkin Elmer) was used. 300 ng of total RNA was subjected to retro-transcription by using the enzyme Reverse Transcriptase (Fermentas).

Semi-quantitative RT-PCR reactions were conducted using the 18S pair/competimer universal primer pair (Ambion) in ratio 6:4 as internal standard. The PCR products were separated on 1.5% agarose gels, visualized using the Geliance instrument (Perkin Elmer) and analyzed by densitometer using the Genetools software.

The values shown in the graphs of Figure 2 represent the ratios between the intensity of the band relative to the analyzed gene and that of the band relative to the reference standard 18S, in order to have a value related to the real expression of that gene and not dependent upon the amount of RNA or PCR reagents present in that sample.

The sequences of the specific primers for the amplification of the CGRP were the following:
hs CGRP b for2: GCAGGTGTGGTGTTCATCCC (Seq. Id. No. 1)
hs CGRP b rev2: GGGCATTCTCACCAAGTTCT (Seq. Id. No. 2)

As shown in Figure 2, the treatment with the extract according to the invention significantly reduced the expression of the CGRP gene at the both used concentrations; in particular, the lowest concentration (0.002%) produced a reduction in gene expression of approximately 65%.

### EXAMPLE 5 - Study of the capacity of C. sativa extract to reduce the inflammation induced by the neuronal peptide CGRP in skin cells.

To determine whether the extract from plant cell cultures according to the invention was able to reduce the inflammation of the skin cells induced by the neurogenic peptide, RT-PCR ("Reverse transcriptase-polymerase chain reaction") experiments were conducted to evaluate the expression of pro-inflammatory cytokines, IL-8 and TNFα, on human keratinocytes treated with the CGRP neuropeptide.

Human keratinocyte cells (HaCat) in an initial number of 15 × 10⁴ per well were grown in 6-well plates in DMEM culture medium (Lonza), supplemented with 10% FBS for 16 hours.

The cells were subsequently treated with the two different concentrations of C. *sativa* extract (0.002% and 0.01% w/v) for 16 h and then subjected to inflammatory stress induced by the presence of the CGRP peptide (1 nM) for 1h and 6h, to measure the expression of the pro-inflammatory cytokine IL-8 and TNFα, respectively. The cells were then collected, and the extracted RNA was retrotranscribed and analyzed by semi-quantitative RT-PCR as described in the previous example, using as internal standard the pair of universal primers 18S pair/competimer (Ambion) in the ratio 4:6 (for IL-8) and 2:8 (for TNFα). The PCR products were analyzed on agarose gel as already described in the previous example.

The sequences of the primers specific for amplification were the following:
hs IL-8 for: GCCACCGGAGCACTCCATAA (Seq. Id. No. 3)
hs IL-8 rev: CTCTTCAAAAACTTCTCCACAACC (Seq. Id. No. 4)
hs TNFα for: ATGAGCACTGAAAGCATGATCC (Seq. Id. No. 5)
hs TNFα rev: TCATACCAGGGCTTGGCCTCA (Seq. Id. No. 6)

As shown in Figure 3, the treatment with 1 nM of CGRP triggered a cascade of biological events that led to inflammation, inducing an increase in the expression of both cytokines compared to untreated control cells.

In particular, the keratinocytes treated for 16 h with the extract of C. *sativa* before the exposure to the neuropeptide showed a reduction in the expression of both the proinflammatory cytokines compared to the negative control sample (exposed to the peptide and not treated with the extract of the present invention): the extract produced a reduction at both the tested doses and on both the analyzed cytokines; in particular, the lower dose caused a reduction of about 25% in the expression of IL-8 and about 50% in TNFα. A similar effect was also produced in the positive control sample, which was exposed to the peptide but previously pretreated with the synthetic compound, TO901317, known for its anti-inflammatory properties.

### EXAMPLE 6 - Measure of histamine content.

To determine whether the plant cell culture extract according to the invention was able to reduce the production of histamine induced by the CGRP peptide in the immune system cells, an assay was conducted for the quantization of the histamine content in macrophages subjected to a stress condition by the CGRP neuropeptide.

RAW 264.7 cells were seeded in 96-well plates in an initial number of 5 x 10⁴, and after 16 h, they were treated with different doses of the extract or with the anti-histamine compound cetirizine dihydrochloride (50 µg/ml), used as a positive control of the experiment. After 2 h of treatment with the extract or with cetirizine, the production of histamine was induced by the treatment with 10 nM of CGRP and, after 16 h, the amount of histamine was measured by adding 100 µl of methanol containing 0.4 M NaOH and 1 mg/ml of o-Phthaldialdehyde (OPT). After 10 minutes of incubation at room temperature, 50 µl of 0.1M HCl was added to each well to stop the reaction. Fluorescence was measured at 443 nm using a 360 nm excitation wavelength from the multi-plate reader, Victor3 (Perkin Elmer). The histamine content was calculated as a percentage of the value of the control cells stressed with 10 nM CGRP, arbitrarily set as 100%.

The results obtained indicated that the treatment of the cells with both concentrations of the extract of the present invention significantly reduced the production of histamine in macrophages similarly to what occurred for cetirizine (Figure 4).

### EXAMPLE 7- Expression analysis of the enzyme responsible for dopamine synthesis, Tyrosine Hydroxylase (TH), and the dopamine transport, VMAT2, in neuronal cells.

In order to analyze the ability of the extract of the present invention to regulate the expression of genes involved in the synthesis and the transport of dopamine in neuronal cells, RT-PCR experiments ("Reverse transcriptase-polymerase chain reaction") were conducted to evaluate the expression of TH and VMAT2.

Neuroblastoma cells (SHSY5Y) were treated as described above in Example 4 and analyzed for the expression of the two genes, using the following specific amplification primers:
hs TH for 2: ACTGCAGCCCCAGCTGCATCCTA (Seq. Id. No. 7)
hs TH rev: AGGTGATGACACTTGTCCAG (Seq. Id. No. 8)
hs VMAT2 for: GAAGCTCATCCTGTTCATCG (Seq. Id. No. 9)
hs VMAT2 rev: ATCAGCAGGAAGGCATAGCT (Seq. Id. No. 10)

As shown in Figure 5, the treatment with the extract significantly stimulated the expression of the analyzed genes at both the used concentrations; in particular, the higher concentration (0.01%) induced the gene expression by about 50% and 70%, respectively of the TH and VMAT2 gene.

### EXAMPLE 8 - Analysis of the effect of C. sativa extract on the expression of the beta endorphin precursor (POMC) in neuronal cells and in human epidermis cells.

In order to analyze the ability of the extract of the present invention to regulate the expression of the beta endorphin precursor (POMC) in neuronal cells and in human epidermis cells, RT-PCR experiments ("Reverse Transcriptase-Polymerase Chain Reaction ") were carried out to evaluate its expression.

Neuroblastoma cells (SHSY5Y) and primary human keratinocytes (NHEK) were treated as described above in Example 4 and analyzed for POMC expression by using the following specific amplification primers:
hs POMC for 2: GGCAAGCGCTCCTACTCCAT (Seq. Id. No. 11)
hs POMC rev 2: TCACTCGCCCTTCTTGTAGG (Seq. Id. No. 12)

As shown in Figures 6A and 6B, the treatment with the extract according to the present invention significantly stimulated the expression of POMC at both the concentrations in neuronal cells (6A) and in primary human keratinocytes (6B): in particular, the lowest concentration (0.002%) induced the expression by about 75% in both neuronal cells and keratinocytes.

## Claims

1. An extract derived from *Cannabis sativa* cell cultures for use in the prevention and treatment of inflammation, in particular neuroinflammation, wherein said extract is obtained by a process comprising the steps of:
a) homogenizing the cell cultures in a hydro-alcoholic solution, said hydro-alcoholic solution being an ethanol solution in water with a concentration of ethanol of from 10% to 98% v/v, thus obtaining a homogenate;
b) separating the solid fraction from the liquid fraction of said homogenate, preferably by centrifugation or filtration;
c) bringing the liquid fraction to dryness, thus obtaining said extract.

2. The extract for the use according to claim 1, wherein said *Cannabis sativa* cells belong to a variety selected from the group comprising *Carmagnola, Santhica 27, Eletta campana* or *Felina 32,* preferably to the *Carmagnola* variety.

3. Cosmetic use of an extract derived from *Cannabis sativa* cell cultures, preferably belonging to a variety selected from the group comprising Carmagnola, *Santhica 27, Eletta campana* or *Felina 32,* more preferably to the *Carmagnola* variety, said extract being obtained by a process comprising the steps of:
a) homogenizing the cell cultures in a hydro-alcoholic solution, said hydro-alcoholic solution being an ethanol solution in water with a concentration of ethanol of from 10% to 98% v/v, thus obtaining a homogenate;
b) separating the solid fraction from the liquid fraction of said homogenate, preferably by centrifugation or filtration;
c) bringing the liquid fraction to dryness, thus obtaining said extract.

4. The cosmetic use according to claim 3, for the treatment of skin blemishes.

5. A process for the preparation of an extract derived from a *Cannabis sativa* cell culture, preferably belonging to a variety selected from the group comprising *Carmagnola, Santhica 27, Eletta campana* or *Felina 32,* more preferably to the *Carmagnola* variety, comprising the steps of:
a) homogenizing the cell culture in a hydro-alcoholic solution, said hydro-alcoholic solution being an ethanol solution in water with a concentration of ethanol of from 10% to 98% v/v, thus obtaining a homogenate;
b) separating the solid fraction from the liquid fraction of said homogenate, preferably by centrifugation or filtration;
c) bringing the liquid fraction to dryness, thus obtaining said extract.

6. The process according to claim 5, wherein the liquid fraction in said step c) is brought to dryness by either evaporation or lyophilization.

7. The process according to claim 5 or 6, wherein said *Cannabis sativa* cell cultures are obtained by cutting out vegetable tissues from *Cannabis sativa* plants, inducing the formation of calli from said tissues on a solid substrate, taking said calli and setting up liquid cell cultures with them.

8. The process according to any one of claims 5-7, wherein the ratio between the volume of said hydro-alcoholic solution and the weight of said culture of *Cannabis sativa* cells is comprised between 5:1 and 2:1 and is preferably 3:1.

9. A composition comprising an extract derived from a culture of *Cannabis sativa* cells obtained by the process according to any one of claims 5-8, and at least a hydrophilic solvent selected from the group comprising water and saline aqueous solutions, or at least an organic solvent selected from the group comprising oils, alcohols, glycerol, organic acids, amides, amines, aldehydes and ketones.

10. The composition according to claim 9, for use in the prevention and treatment of inflammation, in particular neuroinflammation.

11. Cosmetic use of the composition according to claim 9.

12. A pharmaceutical formulation comprising the extract for the use according to any of claims 1-2, or a composition for the use according to claim 10, and a pharmaceutically acceptable vehicle.

13. A cosmetic formulation comprising the extract according to claim 3, or a composition according to claim 11, and a cosmetically acceptable vehicle.

14. A formulation of dietary supplement comprising an extract for the use according to any of claims 1-2, or a composition for the use according to claim 10, and a nutraceutically acceptable vehicle.

## Patentansprüche

1. Extrakt, der aus Zellkulturen von Cannabis sativa gewonnen wurde, zur Verwendung bei der Prävention und Behandlung von Entzündungen, insbesondere von Neuroinflammationen, wobei der Extrakt durch ein Verfahren erhalten wird, das folgende Schritte aufweist:
a) Homogenisieren der Zellkulturen in einer hydro-alkoholischen Lösung, wobei die hydro-alkoholische Lösung eine Ethanol-Lösung in Wasser mit einer Ethanolkonzentration von 10% bis 98% v/v ist, wodurch ein Homogenat erhalten wird;
b) Abtrennen der festen Fraktion von der flüssigen Fraktion des Homogenats, bevorzugt durch Zentrifugation oder Filtration;
c) in den trockenen Zustand bringen der flüssigen Fraktion, wodurch der Extrakt erhalten wird.

2. Extrakt zur Verwendung nach Anspruch 1, wobei die Zellen von Cannabis sativa zu einer Sorte gehören, die ausgewählt ist aus der Gruppe, die Carmagnola, Santhica 27, Eletta campana oder Felina 32 aufweist, bevorzugt zur der Sorte Carmagnola.

3. Kosmetische Verwendung eines Extrakts, der aus Zellkulturen von Cannabis sativa gewonnen wurde, die bevorzugt zu einer Sorte gehören, die ausgewählt ist aus der Gruppe, die Carmagnola, Santhica 27, Eletta campana oder Felina 32 aufweist, bevorzugter zu der Sorte Carmagnola, wobei der Extrakt durch ein Verfahren erhalten wird, das folgende Schritte aufweist:
a) Homogenisieren der Zellkulturen in einer hydro-alkoholischen Lösung, wobei die hydro-alkoholische Lösung eine Ethanol-Lösung in Wasser mit einer Ethanolkonzentration von 10% bis 98% v/v ist, wodurch ein Homogenat erhalten wird;
b) Abtrennen der festen Fraktion von der flüssigen Fraktion des Homogenats, bevorzugt durch Zentrifugation oder Filtration;
c) in den trockenen Zustand bringen der flüssigen Fraktion, wodurch der Extrakt erhalten wird.

4. Kosmetische Verwendung nach Anspruch 3 zur Behandlung von Hautunreinheiten.

5. Verfahren zur Herstellung eines Extrakts, der aus einer Zellkultur von Cannabis sativa gewonnen wird, die bevorzugt zu einer Sorte gehört, die ausgewählt wird aus der Gruppe, die Carmagnola, Santhica 27, Eletta campana oder Felina 32 aufweist, bevorzugter zu der Sorte Carmagnola, folgende Schritte aufweisend:
a) Homogenisieren der Zellkultur in einer hydro-alkoholischen Lösung, wobei die hydro-alkoholische Lösung eine Ethanol-Lösung in Wasser mit einer Ethanolkonzentration von 10% bis 98% v/v ist, wodurch ein Homogenat erhalten wird;
b) Abtrennen der festen Fraktion von der flüssigen Fraktion des Homogenats, bevorzugt durch Zentrifugation oder Filtration;
c) in den trockenen Zustand bringen der flüssigen Fraktion, wodurch der Extrakt erhalten wird.

6. Verfahren nach Anspruch 5, wobei die flüssige Fraktion in dem Schritt c) entweder durch Eindampfen oder Lyophilisieren in den trockenen Zustand gebracht wird.

7. Verfahren nach Anspruch 5 oder 6, wobei die Zellkulturen von Cannabis sativa erhalten werden durch Herausschneiden von Pflanzengeweben aus Cannabis sativa-Pflanzen, Induzieren der Bildung von Kalli aus den Geweben auf einem festen Substrat, Entnehmen der Kalli und Anlegen von Zellen-Flüssigkulturen mit ihnen.

8. Verfahren nach einem der Ansprüche 5 - 7, wobei das Verhältnis zwischen dem Volumen der hydro-alkoholischen Lösung und dem Gewicht der Kultur von Cannabis sativa-Zellen zwischen 5:1 und 2:1 liegt, und bevorzugt 3:1 ist.

9. Zusammensetzung aufweisend einen aus einer Kultur von Cannabis sativa-Zellen gewonnenen Extrakt, der durch das Verfahren nach einem der Ansprüche 5 - 8 erhalten wurde, und mindestens ein hydrophiles Lösungsmittel, das ausgewählt ist aus der Gruppe, die Wasser und wässrige Salzlösungen aufweist, oder mindestens ein organisches Lösungsmittel, das ausgewählt ist aus der Gruppe, die Öle, Alkohole, Glycerol, organische Säuren, Amide, Amine, Aldehyde und Ketone aufweist.

10. Zusammensetzung nach Anspruch 9 zur Verwendung bei der Prävention und Behandlung von Entzündungen, insbesondere Neuroinflammationen.

11. Kosmetische Verwendung der Zusammensetzung nach Anspruch 9.

12. Pharmazeutische Formulierung aufweisend den Extrakt zur Verwendung nach einem der Ansprüche 1 - 2, oder eine Zusammensetzung zur Verwendung nach Anspruch 10, und eine pharmazeutisch annehmbare Trägersubstanz.

13. Kosmetische Formulierung aufweisend den Extrakt nach Anspruch 3 oder eine Zusammensetzung nach Anspruch 11, und eine kosmetisch annehmbare Trägersubstanz.

14. Formulierung eines Nahrungsergänzungsmittels aufweisend einen Extrakt zur Verwendung nach einem der Ansprüche 1 - 2, oder eine Zusammensetzung zur Verwendung nach Anspruch 10, und eine nutrazeutisch annehmbare Trägersubstanz.

## Revendications

1. Un extrait issu de cultures cellulaires de *Cannabis sativa* destiné à être utilisé dans la prévention et le traitement de l'inflammation, en particulier de la neuro-inflammation, ledit extrait étant obtenu par un procédé comprenant les étapes consistant à :
a) homogénéiser les cultures cellulaires dans une solution hydroalcoolique, ladite solution hydroalcoolique étant une solution d'éthanol dans l'eau avec une concentration en éthanol allant de 10% à 98% v/v, de façon à obtenir un homogénat ;
b) séparer la fraction solide de la fraction liquide dudit homogénat, de préférence par centrifugation ou filtration ;
c) amener à sec la fraction liquide, de façon à obtenir ledit extrait.

2. L'extrait pour l'utilisation selon la revendication 1, dans lequel lesdites cellules de *Cannabis sativa* appartiennent à une variété choisie dans le groupe comprenant *Carmagnola, Santhica 27, Eletta campana* ou *Felina 32,* de préférence à la variété *Carmagnola.*

3. Utilisation cosmétique d'un extrait issu de cultures cellulaires de *Cannabis sativa,* appartenant de préférence à une variété choisie dans le groupe comprenant *Carmagnola, Santhica 27, Eletta campana* ou *Felina 32,* de façon encore préférée à la variété *Carmagnola,* ledit extrait étant obtenu par un procédé comprenant les étapes consistant à :
a) homogénéiser les cultures cellulaires dans une solution hydroalcoolique, ladite solution hydroalcoolique étant une solution d'éthanol dans l'eau avec une concentration en éthanol de 10% à 98% v/v, de façon à obtenir un homogénat ;
b) séparer la fraction solide de la fraction liquide dudit homogénat, de préférence par centrifugation ou filtration ;
c) amener à sec la fraction liquide, de façon à obtenir ledit extrait.

4. L'utilisation cosmétique selon la revendication 3, pour le traitement des imperfections cutanées.

5. Un procédé de préparation d'un extrait issu d'une culture cellulaire de *Cannabis sativa,* appartenant de préférence à une variété choisie dans le groupe comprenant *Carmagnola, Santhica 27, Eletta campana* ou *Felina 32,* de façon encore préférée à la variété *Carmagnola,* comprenant les étapes consistant à :
a) homogénéiser la culture cellulaire dans une solution hydroalcoolique, ladite solution hydroalcoolique étant une solution d'éthanol dans l'eau avec une concentration en éthanol allant de 1,10% à 98% v/v, de façon à obtenir un homogénat ;
b) séparer la fraction solide de la fraction liquide dudit homogénat, de préférence par centrifugation ou filtration ;
c) amener à sec la fraction liquide, de façon à obtenir ledit extrait.

6. Le procédé selon la revendication 5, dans lequel la fraction liquide de ladite étape c), est amenée à sec soit par évaporation, soit par lyophilisation.

7. Le procédé selon la revendication 5 ou la revendication 6, dans lequel lesdites cultures de cellules de *Cannabis sativa* sont obtenues en découpant des tissus végétaux de plantes de *Cannabis sativa,* en induisant la formation de cals à partir desdits tissus sur un substrat solide, en prélevant lesdits cals et en mettant en oeuvre des cultures de cellules liquides avec eux.

8. Le procédé selon l'une quelconque des revendications 5 à 7, dans lequel le rapport entre le volume de ladite solution hydroalcoolique et le poids de ladite culture de cellules de *Cannabis sativa* est compris entre 5:1 et 2:1 et est de préférence de 3:1.

9. Une composition comprenant un extrait issu d'une culture de cellules de *Cannabis sativa* obtenu par le procédé selon l'une quelconque des revendications 5 à 8, et au moins un solvant hydrophile choisi dans le groupe comprenant l'eau et les solutions aqueuses salines, ou au moins un solvant organique choisi dans le groupe comprenant les huiles, les alcools, le glycérol, les acides organiques, les amides, les amines, les aldéhydes et les cétones.

10. La composition selon la revendication 9, destinée à être utilisée dans la prévention et le traitement -de l'inflammation, notamment de la neuro-inflammation.

11. Utilisation cosmétique de la composition selon la revendication 9.

12. Une formulation pharmaceutique comprenant l'extrait pour l'utilisation selon l'une quelconque des revendications 1 à 2, ou une composition pour l'utilisation selon la revendication 10, et un véhicule pharmaceutiquement acceptable.

13. Une formulation cosmétique comprenant l'extrait selon la revendication 3, ou une composition selon la revendication 11, et un véhicule cosmétiquement acceptable.

14. Une formulation de complément alimentaire comprenant un extrait pour une utilisation selon l'une quelconque des revendications 1 à 2, ou une composition pour une utilisation selon la revendication 10, et un véhicule nutraceutiquement acceptable.
